# EUROPEAN PATENT APPLICATION

(11) **EP 1 731 184 A1**
(43) Date of publication of application: **13.12.2006**
(21) Application number: 04734499.9
(22) Date of filing: 24.05.2004
(51) Int. Cl.: A61M 5/14

(54) **A INFUSION BAG INCLUDING A MIXING MEDICINE NOZZLE WITH PUNCTURE FUNCTION**

(30) Priority: 18.03.2004 CN 200410022990
(71) Applicant: Hunan Chinasun Pharmaceutical Machinery Co, Ltd, Hunan 410100 (CN)
(72) Inventor: LIU, Xianghua Panpan Road Economic and, Hunan 410100 (CN); PENG, Xunde Panpan Road Economic and, Hunan 410100 (CN)
(74) Representative: Reinhardt, Markus
(86) International application number: PCT/CN2004/000530
(87) International publication number: WO 2005/087293

(57) **Abstract**

Infusion bags including a mixing medicine nozzle with puncture functionality and methods of using the same are disclosed. An example infusion bag comprises: a bag body having a medicine containing chamber; a medicine mixing tube having a first end in communication with the medicine containing chamber of the bag body; and a medicine output nozzle in communication with the medicine containing chamber of the bag body via a medicine output tube. A second end of the medicine mixing tube is connected to a medicine mixing nozzle, a bottom of the medicine mixing nozzle is covered with a protection membrane, and a needle tube in communication with the medicine mixing tube is disposed in an inner chamber of the medicine mixing nozzle.

## Description

### FIELD OF THE DISCLOSURE

The present invention relates to the field of medicine packaging, and more particularly to an improvement to medicine bags for infusion.

### BACKGROUND

Medicine mixing methods in current clinical use include the following three modes. In a first mode, a medicine output nozzle and a medicine mixing nozzle are provided on a bag. At the time of mixing the medicine, an injector is used to draw out the medicinal liquid from the bag, and then to inject the medicinal liquid into a cillin bottle containing powdery or aqueous medicinal preparations. The above procedure is repeated until there is enough medicinal liquid in the cillin bottle. The bottle is subsequently repetitively shaken until the medicine in the bottle is evenly mixed. Finally, the medicine in the bottle is drawn out by the injector and then injected into an infusion bag. This procedure repeats several times until the medicine in the bottle is entirely drawn out. This mode of medicine mixing is currently used in most clinical applications. In a second mode, an independent medicine mixing nozzle is used, which consists of a connection sheath and a needle tube. When in use, the needle tube of the medicine mixing nozzle is first inserted into the cillin bottle, and the other end of the needle tube is inserted into the medicine mixing nozzle on the infusion bag. Because the needle tube is not very tightly connected to the medicine mixing nozzle on the infusion tube, and also because the inner aperture of the needle tube is very small, the medicinal liquid tends to leak along the outer wall of the needle tube due to heavy pressure when the infusion bag is pressed against, thereby contaminating the operating hand and the infusion bag and hence encumbering use of the bag. Therefore, this method is seldom used. In a third mode, an infusion bag having a medicine mixing nozzle is used, which infusion bag mainly comprises a bag body, a medicine output nozzle and a medicine mixing nozzle. Since its medicine mixing nozzle is configured to be in an open state without sealing, if the medicine mixing nozzle is contaminated before use, especially contaminations in corner areas of the needle tube, disinfection is impossible, thereby rendering it impossible to widely use such a method.

### SUMMARY OF THE INVENTION

The technical problem to be solved by the present invention is to overcome the aforementioned defects in the prior art, whereby provides an infusion bag that is directly communicative with a cillin bottle without any contamination by means of a medicine mixing nozzle having a puncture function.

The technical problem of this invention is solved by the technical solution as discussed below. An infusion bag comprises a bag body, a medicine mixing tube with one end thereof in communication with a medicine containing chamber of the bag body, and a medicine output nozzle also in communication with the medicine containing chamber of the bag body via a medicine output tube; the infusion bag is characterized in that the other end of the medicine mixing tube is connected to the medicine mixing nozzle, and, at the bottom of the medicine mixing nozzle, it is covered with a protection membrane, and in inner chamber of the medicine mixing nozzle is disposed a needle tube communicable with the medicine mixing tube; and a clamp is disposed on the medicine mixing tube for opening and closing thereof. The medicine mixing nozzle according to this invention can be comprised of a protection membrane, a connection sheath, a sealing nozzle and a needle tube, wherein the protection membrane covers the bottom of the connection sheath, the sealing nozzle locates is disposed at an upper end of the connection sheath, and the needle tube is disposed inside an inner chamber of the connection sheath and connected to the sealing nozzle, on whose juncture are processed with creases. The medicine mixing nozzle can alternatively be comprised of a protection membrane, a connection sheath, an inner plug and a needle tube, wherein the needle tube is placed inside an inner chamber of the connection sheath, the inner plug sheathes the needle tube, and the protection membrane covers the bottom of the connection sheath.

This invention includes the following technical effects: (1) as a medicine mixing nozzle with puncture function is provided on the infusion bag, a disposable injector and cotton swabs for sanitizing are not needed in the medicine mixing procedure, which saves cost, reduces secondary pollution of medicinal liquid, and simultaneously simplifies the medicine mixing procedure, makes the medicine mixing operation convenient and easy; (2) the medicine mixing nozzle and the infusion bag are formed integrally, so the leak phenomenon when an independent medicine mixing nozzle and an infusion bag are used simultaneously is avoided; and (3) after the medicinal liquid is prepared, the cillin bottle can still hang on the infusion bag, and the security of medicine using and credibility may be enhanced by this clearance.

### BRIEF DESCRIPTION OF THE DRAWINGS

Fig. 1 is a structural illustration of the present invention;
Fig. 2 illustrates the structure of a first example of the medicine mixing nozzle according to the present invention;
Fig. 3 illustrates the structure of a second example of the medicine mixing nozzle according to the present invention; and
Fig. 4 illustrates the structure of a third example of the medicine mixing nozzle according to the present invention.

In the drawings, the following reference numerals refer to the following structures:
1. bag body
2. medicine output nozzle
3. medicine mixing nozzle
4. clamp
5. medicine mixing tube
6. needle tube
7. medicine output tube
8. protection membrane
9. connection sheath
10. sealing nozzle
11. inner plug

### DETAILED DESCRIPTION

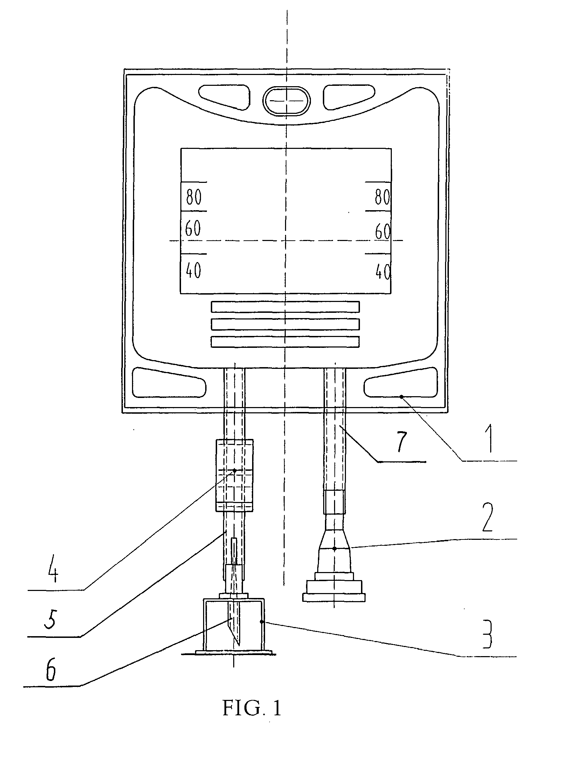

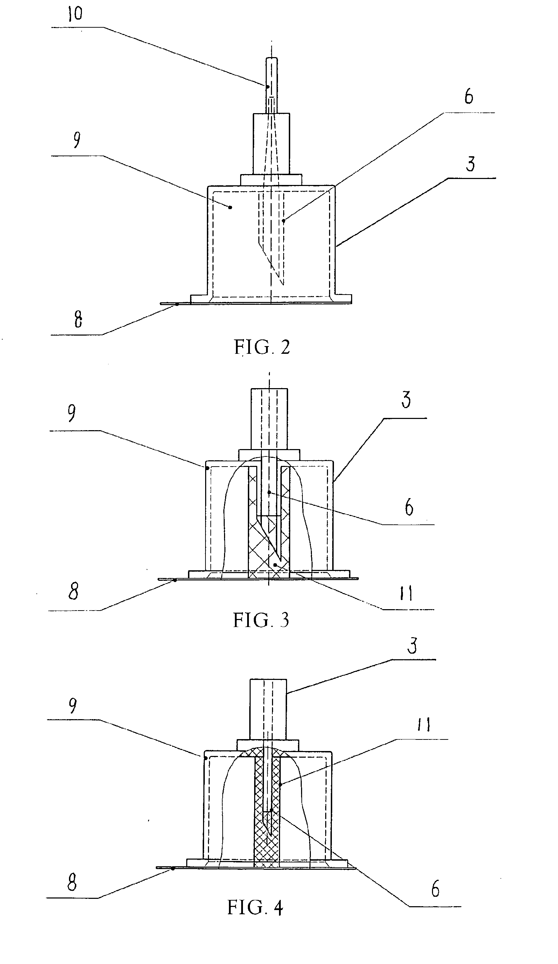

As shown in Fig. 1, the present invention mainly comprises a bag body 1, a medicine output nozzle 2, a medicine mixing tube 5 and a medicine output tube 7.; The medicine mixing tube 5 communicates with its one end with the medicine containing chamber of the bag body 1. The medicine output nozzle 2 also communicates with its one end with the medicine containing chamber of the bag body 1 via the medicine output tube 7. The other end of the medicine output nozzle 2 is sealed. It is characterized in that the other end of the medicine mixing tube 5 is connected to a medicine mixing nozzle 3, whose lower end is configured to be a cylindrical structure with the periphery sealed to facilitate insertion of a cillin bottle nozzle and also to avoid contamination. A needle tube 6 is disposed inside the inner chamber of the cylindrical body of the medicine mixing nozzle 3. The needle tube 6 is communicable with the medicinal liquid chamber via the medicine mixing tube 5. The needle tube 6 can puncture into the cillin bottle in order to draw medicinal liquid when in use to subsequently put the latter into the medicine chamber. The lower end face of the medicine mixing nozzle 3 is covered with a protection membrane 8, which seals the inner chamber of the medicine mixing nozzle 3 to prevent the needle tube 6 from being contaminated during transportation and packaging.

To prevent medicinal liquid from being spilt out of the medicine mixing nozzle 3 during infusion, a clamp 4 is disposed on the medicine mixing tube 5 to effect opening and closing of the passage of the medicine mixing tube 5. The medicine mixing nozzle 3 according to the present invention can be configured as shown in Fig. 2, which shows that the medicine mixing nozzle 3 comprises a protection membrane 8, a connection sheath 9, a sealing nozzle 10 and a needle tube 6, wherein the protection membrane 8 covers the bottom of the connection sheath 9. The sealing nozzle 10 is fixed on an upper end of the connection sheath 9. The needle tube 6 is disposed inside an inner chamber at a lower end of the connection sheath 9. The needle tube 6 is connected to the sealing nozzle 10 with its upper end. The juncture between the needle tube 6 and the sealing nozzle 10 is processed with creases. When in use the juncture connecting the sealing nozzle 10 and the needle tube 6 is first broken by folding so that the needle tube 6 is in communication with its two ends, thereby making it possible for the medicinal liquid in the inner chamber of the cylindrical body of the medicine mixing nozzle 3 to be entirely injected into the infusion bag. The connection sheath 9, the sealing nozzle 10 and the needle tube 6 can be integrally formed by one injection molding, so as to avoid leakage at the positions of the interfaces and contamination during assemblage.

As shown in Fig. 3, the medicine mixing nozzle 3 according to this invention can alternatively comprise a protection membrane 8, a connection sheath 9, an inner plug 11 and a needle tube 6, wherein the needle tube 6 is placed inside an inner chamber at a lower end of the connection sheath 9, the inner plug 11 sheathes the needle tube 6 to while protect the latter and while prevent it from being contaminated, and the protection membrane 8 likewise covers the bottom of the connection sheath 9, thus avoiding contamination of the entire inner chamber of the connection sheath 9. The connection sheath 9 and the needle tube 6 can be constructed as an integral piece by means of one injection molding. When in use, it suffices to first tear out the protection membrane 8 and then pull out the inner plug 11. In comparison with the medicine mixing nozzle 3 as shown in Fig. 3, it is more convenient and reliable in use.

The medicine mixing nozzle 3 of this invention can alternatively be configured as shown in Fig. 4. In the example of FIG. 4, the nozzle 3 can also comprise a protection membrane 8, a connection sheath 9, an inner plug 11 and a needle tube 6. The needle tube 6 may be made of a stainless steel material and may be integrally formed with the connection sheath 2 via one injection molding. When in use it also suffices to pull out the inner plug 3.

The method of using the present invention is: in medicine mixing, the protection membrane 8 on the medicine mixing nozzle 3 is first removed. The needle tube 6 on the medicine mixing nozzle 3 then punctures the rubber plug of the cillin bottle to place the infusion bag in communication with the cillin bottle. Subsequently, the infusion bag is placed higher than the cillin bottle and is repetitively pressed against by hand until the medicinal liquid in the cillin bottle is sufficient to evenly mix the medicine in the bottle. Then the cillin bottle is placed higher than the infusion bag to repeat the aforementioned action until all medicinal liquid in the bottle is drawn back into the infusion bag. Finally, the clamp 4 is used to clamp tightly against the passage of the medicine mixing tube 5 to close it, thus finishing the medicine mixing procedure.

## Claims

1. An infusion bag having a medicine mixing nozzle with puncture function, comprising:
a bag body (1)
a medicine mixing tube (5) in communication with its one end with a medicine containing chamber of the bag body (1), and
a medicine output nozzle (2) also in communication with the medicine containing chamber of the bag body (1) via a medicine output tube(7);
**Characterized in that**, the other end of the medicine mixing tube (5) is connected to a medicine mixing nozzle (3), and the bottom of the medicine mixing nozzle (3) is covered with a protection membrane (8), and in inner chamber of the medicine mixing nozzle (3) is disposed a needle tube (6) in communication with the medicine mixing tube (5).

2. The infusion bag having a medicine mixing nozzle with puncture function according to claim 1, **characterized in that** a clamp (4) is disposed on the medicine mixing tube (5).

3. The infusion bag having a medicine mixing nozzle with puncture function according to claim 1 or 2, **characterized in that**, the medicine mixing nozzle (3) comprises a protection membrane (8), a connection sheath (9), a sealing nozzle (10) and a needle tube (6), wherein the protection membrane (8) covers the bottom of the connection sheath (9), the sealing nozzle (10) is located at an upper end of the connection sheath (9), and the needle tube (6) is disposed inside an inner chamber of the connection sheath (9) and connected to the sealing nozzle (10), and a juncture between the connection sheath and the sealing nozzle is creased.

4. The infusion bag having a medicine mixing nozzle with puncture function according to claim 1 or 2, **characterized in that** the medicine mixing nozzle (3) comprises a protection membrane (8), a connection sheath (9), an inner plug (11) and a needle tube (6), wherein the needle tube (6) is disposed inside an inner chamber of the connection sheath (9), the inner plug (11) sheathes the needle tube (6), and the protection membrane (8) covers the bottom of the connection sheath (9).
